(19) Europäisches Patentamt
European Patent Office
Office européen des brevets

(11) **EP 3 848 465 A1**

## (12) EUROPEAN PATENT APPLICATION
published in accordance with Art. 153(4) EPC

(43) Date of publication:
**14.07.2021 Bulletin 2021/28**

(21) Application number: **19857204.2**

(22) Date of filing: **04.09.2019**

(51) Int Cl.:
***C12P 7/64*** *(2006.01)*

(86) International application number:
**PCT/JP2019/034729**

(87) International publication number:
**WO 2020/050304 (12.03.2020 Gazette 2020/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **04.09.2018 JP 2018165450**

(71) Applicant: **NIPPON SUISAN KAISHA, LTD.
Minato-ku
Tokyo 105-8676 (JP)**

(72) Inventors:
• **HIROUCHI, Yuuji**
  **Hachioji-shi, Tokyo 192-1204 (JP)**
• **YAMAZAKI, Isao**
  **Hachioji-shi, Tokyo 192-1204 (JP)**
• **FURIHATA, Kiyomi**
  **Hachioji-shi, Tokyo 192-1204 (JP)**
• **DOISAKI, Nobushige**
  **Tsukuba-shi, Ibaraki 300-2611 (JP)**

(74) Representative: **Hoffmann Eitle
Patent- und Rechtsanwälte PartmbB
Arabellastraße 30
81925 München (DE)**

(54) **PRODUCTION METHOD FOR DOCOSAHEXAENOIC ACID-CONTAINING GLYCERIDE USING A LIPASE HYDROLYSIS REACTION**

(57) The present invention provides a method of producing a composition containing docosahexaenoic acid as a constituent fatty acid of glycerides, comprising hydrolyzing a feedstock oil containing glycerides comprising docosahexaenoic acid as a constituent fatty acid by action of a *Candida cylindracea* derived lipase and a partial glyceride lipase, thereby increacing the proportion of docosahexaenoic acid in glyceride fractions, wherein the lipase hydrolysis reaction is performed at a temperature no higher than 30°C for a period no longer than 15 hours.

EP 3 848 465 A1

## Description

TECHNICAL FIELD

The present invention relates to a method of producing docosahexaenoic acid enriched glycerides by making use of lipase hydrolysis reaction.

BACKGROUND ART

It has been reported that n-3 highly unsaturated fatty acids such as eicosapentaenoic acid (EPA) and docosahexaenoic acid (DHA) have various physiological actions such as lowering neutral fats in the blood, reducing the risk for heart diseases, and anti-inflammation. Not only n-3 highly unsaturated fatty acids but also n-6 highly unsaturated fatty acids (linoleic acid and arachidonic acid) are essential fatty acids and their adequate intakes have been specified (Dietary Reference Intakes for Japanese (2015)). Since n-6 fatty acids are readily ingested from vegetable oils and meats, it has also been reported that in the western diet, they increase in the human body due to excessive intake and promote inflammation to thereby present a risk for consumers to become vulnerable to arteriosclerosis. In contrast, the intake of fish and seashells is on a downward trend and the intake of n-3 highly unsaturated fatty acids tends to be insufficient. According to the data published in the 2013 National Health and Nutrition Survey, the daily intake of fish and seashells has decreased in all age groups, testifying that Japanese people are losing preference for fish.

To make up for the decreased intake of n-3 highly unsaturated fatty acids, consumers increasingly rely on EPA and DHA supplements. Currently available supplements include refined fish oils on their own as well as concentrated glycerides that typically contain EPA and DHA as enriched from fish oils.

Lipases are enzymes that catalyze the reaction by which oils and fats are hydrolyzed to free fatty acids and glycerol and it is known that various animals and plants as well as microorganisms possess lipases. Certain types of lipases do not equally act on all fatty acids and their reactivity differs with such factors as the binding position on glycerides, the carbon chain length of fatty acids and the number of double bonds they have. Such lipases can therefore be used for selective hydrolysis of fatty acids and, as the result, it is possible to enrich particular fatty acids in glyceride fractions. To give an example, the technique of enriching highly unsaturated fatty acids in glyceride fractions by means of a lipase derived from *Candida cylindracea* has been known from a long time ago (PTL 1).

The lipase-based hydrolysis reaction is an effective method for enrichment of highly unsaturated fatty acids. The more hydrolyzed are fatty acids other than the highly unsaturated fatty acids of interest, the more enriched are the highly unsaturated fatty acids in glyceride fractions. However, enzymatic reactions are equilibrium reactions, so it is not easy to enrich the highly unsaturated fattty acids to more than a certain level. Accordingly, if enrichment to high enough concentrations is desired, measures such as repeating the lipase reaction are taken.

Methods of using two types of lipase in combination to enrich highly unsaturated fatty acids in glyceride fractions have also been reported. For example, PTL 2 discloses that by using two types of lipase in combination as selected from *Candida cylindracea* derived lipase, *Rhizopus* derived lipase, and *Chromobacterium* lipase, glycerides are obtianed that have a higher DHA content than in the case where the respective lipases are used independently. What is more, PTL 3 discloses that by using *Mucor miehei* derived lipase, *Candida cylindracea* derived lipase or *Rhizopus oryzae* derived lipase in combination with *Penicillium camenberti* derived lipase, DHA is enriched in glyceride fractions with improved selectivity, whereby oils or fats can be produced that contain highly unsaturated fatty acids at an increased ratio of DHA to EPA.

CITATION LIST

PATENT LITERATURE

PTL 1: JP S58-165796 A
PTL 2: JP H7-268382 A
PTL 3: WO 98/18952

SUMMARY OF INVENTION

TECHNICAL PROBLEM

Lipase reaction can be performed at mild temperatures near room temperature and although this is a great advantage it has over chemical methods, the reaction time tends to be prolonged. In order to shorten the reaction time, more enzymes need be used. However, enzymes are expensive and increasing their use may potentially result in lowered economy.

Therefore, an object of the present invention which relates to a method of enriching n-3 highly unsaturated fatty acids in glyceride fractions by using the lipase reaction is to provide a novel means for shortening the reaction time while suppressing the enzyme from being used in an increased amount.

SOLUTION TO PROBLEM

The present inventors conducted an intensive study with a view to attaining the above-stated object and have found that by combining two or more particular types of lipase, the time of lipase-assisted hydrolysis reaction can be considerably shortened.

Based on the above-mentioned finding, the present inventors continued their study and eventually accomplished the present invention.

Briefly, the present invention provides the following [1] to [11].

[1] A method of producing a composition containing docosahexaenoic acid as a constituent fatty acid of glycerides, comprising:

hydrolyzing a feedstock oil containing glycerides comprising docosahexaenoic acid as a constituent fatty acid by action of a *Candida cylindracea* derived lipase and a partial glyceride lipase, thereby increacing the proportion of docosahexaenoic acid in glyceride fractions;
wherein the lipase hydrolysis reaction is performed at a temperature no higher than 30°C for a period no longer than 15 hours.

[2] The method as recited in [1], wherein the hydrolysis reaction time required for the proportion of docosahexaenoic acid in fatty acid composition of the glyceride fractions to become 47 area% and above is shortened compared to the case where the partial glyceride lipase is not used.

[3] The method as recited in [1] or [2], wherein the partial glyceride lipase is a *Penicillium camenberti* derived lipase.

[4] The method as recited in any one of [1] to [3], wherein the amount in which the *Candida cylindracea* derived lipase is used ranges from 200 to 1,600 units per gram of the feedstock oil.

[5] The method as recited in any one of [1] to [4], wherein the amount in which the partial glyceride lipase is used ranges from 0.1 to 1 in terms of activity ratio relative to the *Candida cylindracea* derived lipase which is taken as 1.

[6] The method as recited in any one of [1] to [5], wherein the lipase hydrolysis reaction is performed at between 10 and 30°C.

[7] The method as recited in any one of [1] to [6], wherein the proportion of docosahexaenoic acid in fatty acid composition of the glyceride fractions after the reaction is no less than 45 area%.

[8] The method as recited in any one of [1] to [7], wherein the proportion of eicosapentaenoic acid in fatty acid composition of the glyceride fractions after the reaction is no less than 5 area%.

[9] The method as recited in any one of [1] to [8], wherein the amount of water to be added to the reaction solution after the lipase hydrolysis reaction ranges from 0.2 to 1.2 g per gram of the feedstock oil.

[10] The method as recited in any one of [1] to [9], wherein the reaction is terminated at the point in time when the acid value of the reaction solution after the lipase hydrolysis reaction has come to lie between 90 and 150.

[11] The method as recited in any one of [1] to [10] which further comprises a step of recovering the glycerides after the reaction.

ADVANTAGEOUS EFFECTS OF INVENTION

According to the method of the present invention which is an improvement of the method for enriching DHA by means of a *Candida cylindracea* derived lipase, DHA can be enriched in glyceride fractions within a shorter time than heretofore required while suppressing the enzyme from being used in an increased amount. In addition, the concentration of EPA in glyceride fractions can be suppressed from decreasing due to the hydrolysis reaction.

BRIEF DESCRIPTION OF DRAWINGS

[Fig. 1] Fig. 1 is a graph showing the proportion (area%) of DHA or DHA+EPA in fatty acid composition of glyceride fractions of the samples obtained by 10- and 24-hour reactions in Example 1, Comparative Example 1 and Comparative Example 2.

[Fig. 2] Fig. 2 is a graph showing the proportion (area%) of DHA or DHA+EPA in fatty acid composition of glyceride fractions of the samples obtained by 10- and 24-hour reactions in Example 3.

DESCRIPTION OF EMBODIMENTS

The present invention relates to a method of producing a composition containing docosahexaenoic acid as a constituent fatty acid of glycerides, comprising hydrolyzing a feedstock oil containing glycerides comprising docosahexaenoic acid as a constituent fatty acid by action of a lipase derived from *Candida cylindracea* (also known as *Candida rugosa)* and a partial glyceride lipase, thereby increasing the proportion of docosahexaenoic acid in glyceride fractions, wherein the lipase hydrolysis reaction is performed at a temperature no higher than 30°C for a period no longer than 15 hours (this method is hereinafter sometimes referred to as the production method of the present invention). By using the partial glyceride lipase in combination with the *Candida cylindracea* derived lipase, the hydrolysis reaction time can be made shorter than in the case where the *Candida cylindracea* derived lipase is used alone and the time it takes to reach the same degree of decomposition (comparable level of acid value) can be halved. What is more, compared to the case of shortening the reaction time by increasing the amount of use of the *Candida cylindracea* derived lipase, the additional use of the partial glyceride lipase enables the reaction time to be shortened by a comparable level using a smaller total amount of lipases.

As used herein, the term "a composition containing docosahexaenoic acid as a constituent fatty acid of glycerides" means a glyceride composition containing glycerides having docosahexaenoic acid bound thereto. Here, the glyceride composition is a composition containing glycerides as main constituents and it may contain glycerides in an amount of, say, no less than 80 mass%, no less than 85 mass%, no less than 90 mass%, no less than 95 mass%, no less than 99 mass%, no less than 99.5 mass%, or no less than 99.9 mass%.

In the present invention, the glycerides include triglycerides, diglycerides, and monoglycerides.

The feedstock oil to be used in the production method of the present invention may be any oil that contains glycerides comprising docosahexaenoic acid as a constituent fatty acid. Exemplary feedstock oils include naturally occurring oils that are known to be rich in docosahexaenoic acid and may specifically be exemplified by sea creature oils (such as sardine oil, tuna oil, bonito oil, krill oil, seal oil, etc.) and microorganism oils (such as oils produced by microorganisms belonging to the genus *Mortierella,* the genus *Penicillium,* the genus *Aspergillus,* the genus *Rhodotorula,* and the genus *Fusarium).* It is preferred to select feedstock oils that comprise a certain amount of docosahexaenoic acid in fatty acids; preferred feedstock oils comprise at least 10 area%, at least 15 area%, or at least 20 area%, of docosahexaenoic acid, with feedstock oils comprising 10 to 35 area%, or 20 to 35 area% being more preferred. In one embodiment, the feedstock oil to be used in the production method of the present invention is fish oil, preferably tuna oil or bonito oil.

In the present invention, these oils may be used either as such or used after they are processed as by refining or enrichment. In the case of fish oil, for example, a whole fish or remnants of fishery processing such as the head, skin, bones or viscera of fish are ground and steamed, then pressed to be separated into stick water (SW) and pressed meal; the oil or fat that is obtained together with the stick water is separated therefrom by centrifugation, whereupon a crude oil is prepared. The crude oil may be degummed, deacidified, decolored, deodorized or otherwise treated to yield a refined fish oil. To refine the crude oil, thin-film distillation (such as molecular distillation or short-path distillation) or alkali deacidification may be employed. In one embodiment, the feedstock oil to be used in the production method of the present invention is a fish oil treated by short-path distillation, preferably tuna oil treated by short-path distillation.

The *Candida cylindracea* derived lipase which is the first lipase to be used in the present invention is a lipase for enriching docosahexaenoic acid in glyceride fractions and being selective for particular fatty acids, it has such properties that it is less active on docosahexaenoic acid in the constituent fatty acids of glycerides, whereby it increases the proportion of docosahexaenoic acid in the constituent fatty acids of glyceride fractions after the hydrolysis reaction. It should be noted here that in this specification, increasing the proportion of docosahexaenoic acid in the constituent fatty acids of glyceride fractions may also be referred to as enriching docosahexaenoic acid in glyceride fractions. The *Candida cylindracea* derived lipase may be exemplified by Lipase OF (Meito Sangyo Co., Ltd.; this enzyme is hereinafter referred to as Lipase OF), and Lipase AY Amono 30SD (Amano Enzyme Inc.)

The amount of use of the first lipase is not particularly limited but it is preferred to add 200 to 1600 units per gram of the feedstock oil (namely, 200 to 1600 units/g oil) to the reaction system. For instance, the first lipase can be used in an amount of 250 to 1600 units, 300 to 1500 units, 300 to 1200 units, 350 to 1000 units, 350 to 900 units or 400 to 800 units per gram of the feedstock oil. As for an immobilized lipase, it can be repeatedly used, so unlike the lipase that is not immobilized and which is added in the required amount, an excess of the immobilized lipase may be added to the reaction system, from which it can be recovered after the reaction for repeated use. Hence, at least the amounts of use that are indicated above with respect to one gram of the feedstock oil may be added to the reaction system; they may be changed to reasonable values depending, for example, on whether the immobilized lipase is used in batch treatment or column treatment, or how many times its use is to be repeated. For example, the immobilized lipase may be used in an amount of 3 to 30% (w/w), or 4 to 25% (w/w), more preferably 5 to 20% (w/w), relative to the feedstock oil. In this connection, one unit of the lipase is the amount of the enzyme that generates one micromole of free fatty acids per minute and which is measured by the method described in 3. Lipolytic Activity Test under "4.03 Digestion Test" of the General Test, Processes and Apparatus in the Japanese Pharmacopoeia, 17th Edition. Specifically, olive oil is used as the substrate. Olive oil is mixed with a liquid emulsifier, whereupon it is emulsified to form a substrate solution. After allowing the lipase to act on the olive oil, sodium hydroxide is added to neutralize the generated fatty acids and the remaining excess of sodium hydroxide is titrated with hydrochloric acid. Titration of sodium hydroxide with hydrochloric acid is also performed on a blank solution prepared without adding the lipase. The lipolytic activity of the lipase (in units/g) is determined from the difference between the two values of titration.

The partial glyceride lipase which is the second lipase to be used in the present invention is preferably a lipase derived from a microorganism of the genus *Penicillium.* As used herein, partial glyceride lipases refer to lipases that hydrolyze monoglycerides and diglycerides but do not hydrolyze triglycerides as well. The lipase derived from a microorganism of the genus *Penicillium* is preferably a lipase derived from *Penicillium camemberti* and may be exemplified by Lipase G Amano 50 (manufactured by Amano Enzyme Inc. and hereinafter referred to as "Lipase G").

The amount of use of the second lipase is not particularly limited but it can be within the range of 0.1 to 1 in terms of activity ratio (the ratio of unit numbers) relative to the first lipase which is taken as 1. Preferably, the second lipase can be used in such amounts that the activity ratio of the first lipase to the second lipase lies between 1:0.15 to 0.85, more preferably between 1:0.2 to 0.75.

In this connection, one unit of the partial glyceride lipase shall be the amount of the enzyme that is measured by the LV emulsification method and which causes a one-micromole increase of fatty acids per minute. The LV emulsification method determines unit numbers by the following procedure: the lipase is acted on an emulsion of vinyl laurate and the reaction is quenched by an ethanol/acetone mixed solvent and after neutralizing the generated fatty acids with sodium hydroxide, the remaining sodium hydroxide is titrated with hydrochloric acid to quantify the generated fatty acids.

In the production method of the present invention, the first lipase and the second lipase are acted on the feedstock oil to perform hydrolysis reaction. The first and the second lipase may both be present within the reaction system from the very beginning of the reaction; alternatively, they may be sequentially supplied into the reaction system with a certain interval provided between the two additions. In the latter case, the second lipase is usually added after the addition of the first lipase. Since the first lipase has difficulty in acting on docosahexaenoic acid in the constituent fatty acids of glycerides, the proportion of docosahexaenoic acid in glyceride fractions can be increased by causing the first lipase to act on the feedstock oil. It should be noted here that since the second lipase is a partial glyceride lipase, it does not directly act on the feedstock oil if the feedstock oil does not contain partial glycerides; in the present invention, however, causing a lipase to act on partial glycerides resulting from the feedstock oil is also included in the concept of causing the lipase to act on the feedstock oil.

Hydrolysis reaction by a lipase need be performed in the presence of a sufficient amount of water for the hydrolytic activity of the lipase to be exhibited. The amount of addition of water ranges from 0.2 to 1.2 parts by weight, preferably from 0.4 to 0.8 parts by weight per part by weight of the feedstock oil.

In order to suppress the deterioration of fatty acids, deactivation of lipases and other deleterious effects, hydrolysis is preferably performed under an inert gas atmosphere such as dry nitrogen. Antioxidants such as tocopherol, ascorbic acid and t-butyl hydroquinone may be contained in the feedstock oil.

The reaction temperature for hydrolysis is not higher than 30°C and it may be the temperature at which lipases exhibit their activity. The reaction temperature is preferably between about 10 and 30°C. To lower the proportion of saturated fatty acids in fatty acid composition of glyceride fractions, the reaction is carried out at no higher than 25°C, say, between 10 and 25°C, preferably between 15 and 25°C. Saturated fatty acids tend to be ingested excessively in the modern diet. Since the docosahexaenoic acid enriched compositions obtained by the production method of the present invention can be used as raw materials for health foods, medicines and other products, they preferably have low contents of saturated fatty acids. The lower the reaction temperature, the lower the content of saturated fatty acids;

however, considering that at 10°C and below, the rate of lipase reaction becomes unduly slow and the viscosity of oils or fats becomes high, the most preferred reaction temperature is in the neighborhood of 15 to 25°C. In the case of high-volume reaction, it may be carried out with the temperature in the reaction vessel being set at values of 15 to 25°C on average while ensuring that changes in the reaction temperature will be held within the range of about ±5°C. Hydrolysis reaction can be performed under flowing conditions created by mechanical agitation or with an inert gas or other media being blown in.

Hydrolysis reaction should be continued until the proportion of docosahexaenoic acid in fatty acid composition of glyceride fractions becomes an intended level. The reaction time can differ with such factors as the feedstock oil and the amount of enzyme. For example, in the case where the first lipase is used in a large amount, the reaction time may be shortened and in the case where the first lipase is used in a small amount, the reaction time may be prolonged. The reaction time is preferably no longer than 15 hours. In one embodiment, the reaction time can, for example, be no less than 7 hours, no less than 8 hours, no less than 9 hours, or no less than 10 hours but no more than 15 hours, no more than 14 hours, no more than 13 hours, no more than 12 hours, or no more than 11 hours. The reaction time can, for example, lie between 8 and 15 hours, or between 10 and 15 hours

According to the production method of the present invention, the proportion of docosahexaenoic acid in glyceride fractions can be increased to 45 area% and above, 46 area% and above, 47 area% and above, 48 area% and above, 49 area% and above, 50 area% and above, or 51 area% and above by a single run of lipase reaction. If, for example, tuna oil (with a DHA content of about 23%) is used as a feedstock, hydrolysis may be performed for 7 hours or longer with the already indicated amounts of enzyme, whereupon the proportion of docosahexaenoic acid can be increased to 45 area% and above by a single run of lipase reaction. Consequently, the lipase-assisted hydrolysis reaction in the production method of the present invention can be completed by a single run of reaction and glycerides containing docosahexaenoic acid in high proportions can be recovered from the reaction solution.

In this specification, unless otherwise noted, fatty acid composition (area%) of glyceride fractions is determined as follows: glycerides in the oil layer of the lipase reaction product are methyl esterified by the sodium methylate method and, thereafter, the free fatty acids generated by the lipase reaction are removed and the residue is subjected to gas chromatographic measurement to give values (peak areas), based on which fatty acid composition (area%) of glyceride fractions is calculated. The conditions for gas chromatographic analysis are as follows.
Device type: Agilent 6890N GC system (Agilent Technologies)
Column: DB-WAX J&W 122-7032
Column temperature: Held at 180°C for 0 minutes, then elevated from 180°C to 230°C at 3°C/min, and held for 15 minutes.
Injection temperature: 250°C
Injection method: Splitting
Split ratio: 30:1
Detector temperature: 250°C
Detector: FID
Carrier gas: Helium (1 mL/min, constant flow)

As docosahexaenoic acid in glyceride fractions is more enriched by the lipase reaction, the proportion of eicosapentaenoic acid in the glyceride fractions is sometimes lowered. According to the production method of the present invention, the proportion of eicosapentaenoic acid in fatty acid composition of glyceride fractions is suppressed from decreasing. In one embodiment, the proportion of eicosapentaenoic acid in fatty acid composition of glyceride fractions after the reaction can be no less than 5 area%, no less than 6 area%, no less than 7 area%, no less than 8 area%, no less than 9 area%, or no less than 10 area%.

What is more, according to the production method of the present invention, the proportion of the sum of docosahexaenoic acid and eicosapentaenoic acid in fatty acid composition of glyceride fractions can be increased to 50 area% and above, 51 area% and above, 52 area% and above, 53 area% and above, 54 area% and above, 55 area% and above, 56 area% and above, 57 area% and above, 58 area% and above, 59 area% and above, or 60 area% and above, by a single run of lipase reaction.

Acid value (AV) serves as an indicator for the degree of hydrolysis. In the present invention, acid value is a numeric value measured by a method as modified from the JOCS Standard Methods for the Analysis of Fats, Oils and Related Materials (2013 Edition) (compiled by Japan Oil Chemists' Society). Specifically, acid value is measured in the following manner: about 100 mg of a sample is dissolved in 20 mL of a neutral solvent (ethanol : diethyl ether = 1:1 v/v) and after adding a phenolphthalein solution, neutralizing titration is performed with a 0.1 M potassium hydroxide standard solution and the acid value of interest is computed by the following formula:

$$AV = \text{amount of titrant (mL)} \times 56.11 \times \text{factor of potassium hydroxide standard solution/sample weight (g)} \times (1/10).$$

In the above formula, "factor of potassium hydroxide standard solution" means "(standardization-determined) true concentration of the standard solution divided by the indicated concentration of the prepared standard solution)."

In the present invention, hydrolysis reaction is usually performed to give an acid value of 90 to 150, whereby the proportion of docosahexaenoic acid in fatty acid composition of glyceride fractions can be adequately increased. If the reaction continues until the acid value exceeds 150, the percent recovery of docosahexaenoic acid is likely to decrease. Hence, in the production method of the present invention, hydrolysis reaction can be terminated at the point in time when the acid value of the reaction solution has come to lie between 100 and 150, or between 110 and 140.

According to the present invention, the hydrolysis reaction time it takes for the proportion of docosahexaenoic acid in fatty acid composition of glyceride fractions to become 47 area% and above is shortened compared to the case where the partial glyceride lipase is not additionally used. In this case, the reference for comparison is the time of reaction using the *Candida cylindracea* derived lipase alone in the same amount as specified for the production method of the present invention. For example, the reaction performed using 200 to 1600 units/g oil of the *Candida cylindracea* derived lipase (the reaction as the reference for comparison) is compared with the reaction performed with the partial glyceride lipase being further added (the reaction performed in the production method of the present invention).

In one embodiment, the present invention ensures that the time the hydrolysis reaction takes for the proportion of docosahexenoic acid in fatty acid composition of glyceride fractions to become 47 area% and above is shortened by no less than 1 hour, no less than 2 hours, no less than 3 hours, no less than 4 hours, no less than 5 hours, no less than 6 hours, no less than 7 hours, no less than 8 hours, no less than 9 hours, no less than 10 hours, no less than 11 hours, no less than 12 hours, no less than 13 hours, or no less than 14 hours, compared to the case where the partial glyceride lipase is not additionally used. Alternatively, the time the hydrolysis reaction takes for the proportion of docosahexenoic acid in fatty acid composition of glyceride fractions to become 47 area% and above is shortened by no less than 10%, no less than 20%, no less than 30%, no less than 40%, no less than 50%, or no less than 55%, compared to the case where the partial glyceride lipase is not additionally used.

Furthermore, according to the present invention, the time the lipase hydrolysis reaction takes for the reaction solution to have an acid value of 110 and above is shortened compared to the case where the partial glyceride lipase is not additionally used. In one embodiment, the present invenion ensures that the time the lipase hydrolysis reaction takes for the reaction solution to have an acid value of 110 and above is shortened by no less than 1 hour, no less than 2 hours, no less than 3 hours, no less than 4 hours, no less than 5 hours, no less than 6 hours, no less than 7 hours, no less than 8 hours, no less than 9 hours, no less than 10 hours, no less than 11 hours, no less than 12 hours, no less than 13 hours, or no less than 14 hours compared to the case where the partial glyceride lipase is not additionally used. Alternatively, the time the lipase hydrolysis reaction takes for the reaction solution to have an acid value of 110 and above is shortened by no less than 10%, no less than 20%, no less than 30%, no less than 40%, no less than 50%, or no less than 55% compared to the case where the partial glyceride lipase is not additionally used.

Advantageous effects of the present invention other than shortening of the reaction time include enhancement of EPA recovery and concentration.

As will be described in Examples, the EPA concentration can be increased to 5 area% and above even if DHA is enriched to a concentration of 50 area% and above. It is also possible to increase the EPA concentration to 5.6 area% and above, 6.0 area% and above, 6.4 area% and above, 6.7 area% and above, or 7.4 area% and above. If DHA is enriched to a concentration of 46 area% and above, the EPA concentration can be increased to 6 area% and above. The EPA concentration can be further increased to 6.5 area% and above, 7.0 area% and above, 7.5 area% and above, 7.9 area% and above, or 8.0 area% and above.

According to the method of the present invention, the selectivity between fatty acids by the overall reaction system will so vary that both the EPA recovery and the EPA concentration can be increased. Since both EPA and DHA are preferred fatty acids which are beneficial for health, the capability of increaicng the percent residues of these fatty acids will provide an advantageous effect, no matter how small the increase may be.

The production method of the present invention may further comprise the step of recovering glycerides after the hydrolysis reaction by lipases. To recover the glycerides, the following procedure may be followed: after the period of hydrolysis reaction ends, the lipases used are deactivated by heating or adding an acid, and the aqueous layer, free fatty acids and glycerol are removed from the reaction solution. The aqueous layer, free fatty acids and glycerol can be removed by known methods. For example, after the period of hydrolysis reaction ends, the lipases used are deactivated by adding an acid; the aqueous layer comprising the lipases and glycerol is removed from the reaction solution as by centrifugation; further, rinsing with water is repeated until the aqueous layer becomes neutral; thereafter, free fatty acids are removed from the resulting oil layer. Free fatty acids can be separated and removed by known methods, for example, removing them in the form of alkali salts, using a liquid chromatographic apparatus, or removing them by distillation; preferred methods are the removal of free fatty acids as alkali salts, and removal by distillation such as molecular distillation or short-path distillation. By removing the free fatty acids, a triglyceride/partial glyceride mixture containing docosahexaenoic acid at high concentration is obtained.

The compositions obtained by the produciton method of the present invention have high proportions of triglerides in glyceride fractions as compared with the case where the *Candida cylindracea* derived lipase is used alone . The glycerides obtrained by hydrolysis with the *Candida cylindracea* derived lipase are inherently characterized by containing a higher proportion of triglycerides than when other lipases are used. As shown in Examples, the proportion of triglycerides in glyceride fractions can be further increased by combining the *Candida cylindracea* derived lipase with the partial glyceride lipase. The proportion of triglycerides increases in proportion to the amount in which the partial glyceride lipase is added. According to the produciton method of the present invention, the proportion of triglycerides in glyceride fractions can be increased by, for example, no less than 1 area%, no less than 2 area%, no less than 3 area%, no less than 4 area%, or no less than 5 area% compared to the case where the partial glyceride lipase is not added. What is more, the composition obtained by the produciton method of the present invention has a low proportion of monoglycerides in glyceride fractions. Consequently, according to the present invention, the recovered glycerides need not be subjected to further removal of partial glycerides and this helps simplify the refining process.

The recovered glycerides can be further subjected to deacidifying, decoloring or deodorizing treatment. Deacidifying, decoloring or deodorizing treatment may be performed by any methods but the deacidifying treatment may be exemplified by distillation treatment, the decoloring treatment may be exemplified by treatment as with activated clay, activated charcoal, or silica gel, and the deodorizing treatment may be exemplified by steam distillation. If the deacidifying treatment is performed by distllation, monoglycerides are removed simultaneously and this contributes to further increasing the ratio of triglycerides in the oil obtained.

In still another embodiment of the present invention, there is provided a method for shortening the duration of reaction in which a feedstock oil containing glycerides comprising docosahexaenoic acid as a constituent fatty acid is subjected to hydrolysis by the *Candida cylindracea* derived lipase. This method includes performing the hydrolysis reaction by allowing the *Candida cylindracea* derived lipase and the partial glyceride lipase to act on the feedstock oil containing glycerides comprising docosahexaenoic acid as a constituent fatty acid.

The feedstock oil, the *Candida cylindracea* derived lipase and the partial glyceride lipase are as already described in connection with the produciton method of the present invention.

According to the present invention, the hydrolysis reaction time it takes for the proportion of docosahexaenoic acid in fatty acid composition of glyceride fractions to become 47 area% and above, or for the acid value of the lipase hydrolysis reaction solution to become 110 and above can be shortened as compared with the case of reaction that uses the same amount of the *Candida cylindracea* derived lipase alone. For example, the raction time can be shortened by no less than an hour, no less than 2 hours, no less than 3 hours, no less than 4 hours, no less than 5 hours, no less than 6 hours, no less than 7 hours, no less than 8 hours, no less than 9 hours, no less than 10 hours, no less than 11 hours, no less than 12 hours, no less than 13 hours, or no less than 14 hours. Alternatively, the reaction time can be shortened by no less than 10%, no less than 20%, no less than 30%, no less than 40%, no less than 50%, or no less than 55%.

The present invention is described by means of the following Examples, to which the present invention is by no means limited.

In each of the Examples, fatty acid composition, lipid composition, and the acid value were measured by the following methods.

In the present invention, area% is the proportion to total peak area of the peak area of each of the components in a chart of analysis conducted by gas chromatography or thin-layer chromatograph/hydrogen flame ionization detector (TLC/FID) on a mixture of glycerides that are composed of various fatty acids; in other words, area% indicates the ratio of the content of the component represented by the peak. The fatty acid composition was computed from the results obtained by gas chromatographic analysis in accordance with the method described in the Examples; the lipid composition was computed from the results of analysis using TLC/FID.

<Measuring fatty acid composition>

Fatty acid composition in a feedstock fish oil was measured by gas chromatography after methyl esterification of the fish oil. To be specific, 1 mL of 1 N sodium methylate/methanol solution was added to 40 $\mu$L of fish oil and the resulting mixture was stirred at 80°C for one minute. Subsequently, 1 mL of 1 N hydrochloric acid was added to neutralize the mixture, to which 2 mL of hexane and 3 mL of a saturated aqueous solution of sodium chloride were added and stirred; after allowing the stirred mixture to settle undisturbed, the upper layer was subjected to measurement by gas chromatography.

The fatty acid composition of the glyceride fractions of an oil subjected to lipase reaction was measured by a process consisting of methyl esterifying the glyceride fractions, removing the free fatty acids generated by lipase reaction, and subjecting the residue to gas chromatography. To be specific, 1 mL of 1 N sodium methylate/methanol solution was added to 70 $\mu$L of the reaction solution and the resulting mixture was stirred at 80°C for one minute. Subsequently, 1 mL of 1 N hydrochloric acid was added to neutralize the mixture, to which 700 $\mu$L of hexane and 3 mL of a saturated aqueous solution of sodium chloride were added and stirred; after allowing the stirred mixture to settle undisturbed, the upper layer containing methyl ester and free fatty acids was recovered. The operation of removing free fatty acids from the obtained upper layer was performed as described below. To 700 μL of the hexane solution which was the recovered upper layer having the methyl ester and free fatty acids dissolved therein, 10-20 μL of trimethylamine was added and mixed under shaking; thereafter, the entire volume of the mixture was loaded on a solid-phase extraction cartridge (Agilent Technology, BOND ELUT SI, 100 mg, 1 mL) and the methyl ester was eluted with a mixed solution of hexane and ethyl acetate (hexane : ethyl acetate = 50:1 in volume ratio) to remove the free fatty acids. The residue was subjected to measurement by gas chromatography.

Conditions for Gas Chromatographic Analysis

Device type: Agilent 6890N GC system (Agilent Technologies)
Column: DB-WAX J&W 122-7032
Column temperature: Held at 180°C for 0 minutes, then elevated from 180°C to 230°C at 3°C/min, and held for 15 minutes.
Injection temperature: 250°C
Injection method: Splitting
Split ratio: 30:1
Detector temperature: 250°C
Detector: FID
Carrier gas: Helium (1 mL/min, constant flow)

<Measurement of acid value (AV)>

Acid value (AV) was measured by a method as modified from the JOCS Standard Methods for the Analysis of Fats, Oils and Related Materials (2013 Edition) (compiled by Japan Oil Chemists' Society). Specifically, about 100 mg of a sample was dissolved in 20 mL of a neutral solvent (ethanol: diethyl ether = 1:1 by volume) and after adding a phenolphthalein solution, neutralizing titration was performed with a 0.1 M potassium hydroxide standard solution and the acid value of interest was computed by the following formula:

$$AV = \text{amount of titrant (mL)} \times 56.11 \times \text{factor of potassium hydroxide standard solution/sample weight (g)} \times (1/10).$$

<Percent recovery>

The percent recovery of DHA into glyceride fractions was calculated by the following formula. The percent recovery of EPA was calculated in the same manner. Saponification value was obtained by calculation based on the average molecular weight of the feedstock oil as determined from the fatty acid composition.

$$\text{DHA recovery} = (\text{DHA area\% in glyceride fractions}) \times (1 - \text{acid value/feedstock's saponification value})/(\text{DHA area\% in feedstock oil}).$$

<Measuring lipid composition>

Lipid composition was measured by thin-layer chromatograph/hydrogen flame ionization detector (TLC/FID, IATROSCAN, Mitsubishi Kagaku Iatron, Inc.). Specifically, 20 μL of an oil was dissolved in 1 mL of hexane and 0.5 μL of the solution was loaded on a Chromarod. A thin layer was developed for 30 minutes using a mixed solution of hexane, diethyl ether, and acetic acid (hexane : diethyl ether : acetic acid = 70:30:1 in volume ratio) as a developing solvent. The developed thin layer was analyzed by IATROSCAN.

EXAMPLES

<Example 1>

To 3 g of a refined fish oil (deacidified tuna oil with 24.6 area% DHA and 6.8 area% EPA), Lipase OF, Lipase G and water were added in respective amounts of 400 units/g oil, 100 units/g oil, and 2 g. Under stirring at 18°C, hydrolysis reaction was performed for 24 hours. After 10 hours and 24 hours, the reaction solution was sampled and 85% phosphoric acid was added in an amount of 1.5% relative to the oil. By subsequent stirring at 18°C for an hour, the lipases were deactivated. By subsequent three rinses with hot water, an oil layer of the lipase reaction solution was obtained. The acid value of the oil layer of the reaction solution, the fatty acid composition of glyceride fractions, and the percent recoveries of EPA and DHA were measured.

<Comparative Examples 1 and 2>

Hydrolysis reaction was performed as in Example 1 except that the combination of Lipase OF and Lipase G was replaced by the sole use of Lipase OF in 400 units/g oil (Comparative Example 1) or 800 units/g oil (Comparative Example 2). Subsequently, the acid values of the reaction solutions and the fatty acid composition of glyceride fractions were measured.

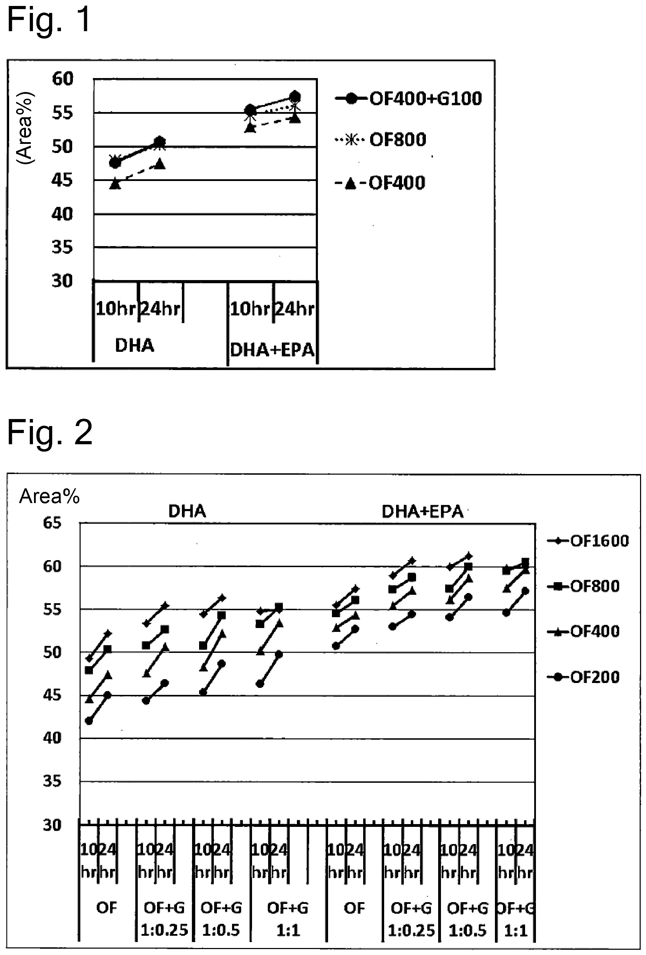

The proportions of DHA and EPA in the fatty acid composition of glyceride fractions are shown in Table 1 and Fig. 1. The percent recoveries of DHA and EPA are shown in Table 2, and the acid values of the reaction solutions are shown in Table 3.

As shown in Table 1 and Fig. 1, it took 24 hours for the concentration of DHA to reach 47 area% in Comparative Example 1 but in Comparative Example 2 where the amount of Lipase OF was doubled and in Example 1 where Lipase OF was combined with Lipase G, 10-hour reaction was sufficient for the concentration of DHA to reach 47 area%. What is more, as shown in Table 3, the acid value that required 24-h reaction to reach in Comparative Example 1 was reached by 10-hour reaction in Example 1.

It should also be noted that in Comparative Examples 1 and 2 using Lipase OF alone, the concentration of EPA started to decrease as more DHA was enriched; however, in Example 1 which used Lipase G in combination with Lipase OF, the concentration of EPA was suppressed from decreasing. As Table 1 and Fig. 1 show, compared to the case of Comparative Example 2 which used Lipase OF alone, Example 1 which used Lipase G in combination with Lipase OF allowed the concentration of EPA to be more effectively suppressed from decreasing when DHA was enriched to a comparable level of concentration.

As for the percent recovery, Example 1 and Comparative Example 2 provided almost comparable levels of DHA recovery after a 10-hr reaction but the recovery of EPA was higher in Example 1 than in Comparative Example 2 (Table 2).

[Table 1]

| Concentration (area%) | Reaction time 10hr | | Reaction time 24hr | |
|---|---|---|---|---|
| | DHA | EPA | DHA | EPA |
| Ex. 1 | 47.6 | 7.9 | 50.6 | 6.7 |
| Com. Ex. 1 | 44.6 | 8.3 | 47.4 | 7.0 |
| Com. Ex. 2 | 47.9 | 6.7 | 50.3 | 5.8 |

[Table 2]

| Recovery (wt%) | Reaction time 10hr | | Reaction time 24hr | |
|---|---|---|---|---|
| | DHA | EPA | DHA | EPA |
| Ex. 1 | 81.8 | 49.1 | 77.8 | 37.4 |
| Com. Ex. 1 | 85.9 | 57.4 | 82.5 | 43.6 |
| Com. Ex. 2 | 81.0 | 40.6 | 79.1 | 32.6 |

[Table 3]

| Acid value | Reaction time | |
|---|---|---|
| | **10hr** | **24hr** |
| Ex. 1 | **114.9** | **123.7** |
| Com. Ex. 1 | **104.5** | **113.8** |
| Com. Ex. 2 | **116.2** | **122.0** |

<Example 2>

The same lipase reaction as in Example 1 and Comparative Examples 1 and 2 was performed at 30°C for 10 hours to give results that were respectively designated as Example 2 and Comparative Examples 3 and 4. Table 4 shows the proportions of DHA and EPA in fatty acid composition of glyceride fractions as obtained by 10-hour reaction.

Even at 30°C, the tendency was similar to that observed in the reaction at 18°C. To be more specific, by using Lipase OF and Lipase G in combination, compared to the case of using Lipase OF alone, the concentration of DHA in glyceride fractious could be more increased by the same reaction time and the concentration of EPA was more effectively suppressed from decreasing when DHA was enriched to a comparable level of concentration.

[Table 4]

| Concentration (area%) | Reaction time 10hr | |
|---|---|---|
| | **DHA** | **EPA** |
| Ex. 2 | **47.5** | **7.3** |
| Com. Ex. 3 | **45** | **7.6** |
| Com. Ex. 4 | **47.4** | **5.9** |

<Example 3>

The same lipase reaction as in Example 1 was performed with the lipase concentration and Lipase G/Lipase OF ratio being varied. To be more specific, the concentration of Lipase OF was set at 200, 400, 800 and 1600 units/g oil, and 0, 0.25, 0.5 or 1 unit of Lipase G was used in combination with one unit of Lipase OF.

Table 5 and Fig. 2 show the proportions of DHA and EPA in fatty acid composition of glyceride fractions after 10- and 24-hr reactions.

Whichever conditions were employed, it was shown that by using Lipase G in combination with Lipase OF, the concentrations of DHA that took 24 hours to reach when the same amounts of Lipase OF were used alone were reached in about 10 hours, namely, the reaction time could be shortened. It was also shown that by using Lipase G in combination with Lipase OF, the concentrations of DHA obtained with two volumes of Lipase OF alone were reached upon reaction for the same periods. It should also be noted that by using Lipase G in combination with Lipase OF, the concentration of EPA was more effectively suppressed from decreasing than in the case where Lipase OF was used alone and, as the result, there were obtained compositions having higher total DHA and EPA concentrations.

[Table 5]

| | DHA | | | | | | | |
|---|---|---|---|---|---|---|---|---|
| | OF | | OF+G 1: 0.25, | | OF+G 1 : 0.5 | | OF+G 1 : 1 | |
| Reaction time | **10hr** | **24hr** | **10hr** | **24hr** | **10hr** | **24hr** | **10hr** | **24hr** |
| **GF1600** | **49.3** | **52.2** | **53.4** | **55.5** | **54.5** | **56.4** | **54.8** | **55.0** |
| **OF800** | **47.9** | **50.3** | **50.8** | **52.7** | **50.8** | **54.3** | **53.3** | **55.3** |
| **OF400** | **44.6** | **47.4** | **47.6** | **50.6** | **48.3** | **52.2** | **50.2** | **53.5** |
| **OF200** | **42.0** | **45.0** | **44.3** | **46.4** | **45.3** | **48.7** | **46.3** | **49.8** |

(continued)

| | EPA | | | | | | | |
| --- | --- | --- | --- | --- | --- | --- | --- | --- |
| | OF | | OF+G 1 : 0.25 | | OF+G 1 : 0.5 | | OF+G 1: 1 | |
| Reaction time | 10hr | 24hr | 10hr | 24hr | 10hr | 24hr | 10hr | 24hr |
| **OF1600** | 6.3 | 5.3 | 5.6 | 5.2 | 5.6 | 5.0 | 5.0 | 5.0 |
| **OF800** | 6.7 | 5.8 | 6.7 | 6.0 | 6.7 | 5.7 | 6.4 | 5.3 |
| **OF400** | 8.3 | 7.0 | 7.9 | 6.7 | 7.9 | 6.5 | 7.4 | 6.2 |
| **OF200** | 8.8 | 7.8 | 8.8 | 8.1 | 8.8 | 7.7 | 8.4 | 7.4 |

In Table 6, data for the proportion of triglycerides in the glyceride fractions of the samples after 10-hr reaction are shown in terms of area%. It can be seen that by using Lipase G in combination with Lipase OF, the proportion of triglycerides increased. The proportion of monoglycerides was low in all cases tested and ranged from 0.1 to 2.1.

[Table 6]

| | OF | OF+G 1 : 0.25 | OF+G 1 : 0.5 | OF+G 1 : 1 |
| --- | --- | --- | --- | --- |
| **DF1600** | 77.3 | 79.2 | 81.5 | 80.3 |
| **OF800** | 77.0 | 78.0 | 81.6 | 82.4 |
| **OF400** | 76.0 | 79.5 | 81.2 | 79.4 |
| **OF200** | 76.5 | 79.2 | 81.7 | 81.7 |

INDUSTRIAL APPLICABILITY

According to the present invention, the lipase reaction time can be shortened to thereby enhance productivity in the method of enriching DHA in glyceride fractions by the *Candida cylindracea* derived lipase. Furthermore, the concentration of EPA in glyceride fractions resulting from the hydrolysis reaction can be suppressed from decreasing, whereby compositions can be obtained that have high total DHA and EPA concentratins. The compositions are particularly useful in the manufacture of compositions that are intended to make use of physiologically active functions common to DHA and EPA, such as neutral fat lowering action.

**Claims**

1. A method of producing a composition containing docosahexaenoic acid as a constituent fatty acid of glycerides, comprising:

   hydrolyzing a feedstock oil containing glycerides comprising docosahexaenoic acid as a constituent fatty acid by action of a *Candida cylindracea* derived lipase and a partial glyceride lipase, thereby increacing the proportion of docosahexaenoic acid in glyceride fractions;
   wherein the lipase hydrolysis reaction is performed at a temperature no higher than 30°C for a period no longer than 15 hours.

2. The method according to claim 1, wherein the hydrolysis reaction time required for the proportion of docosahexaenoic acid in fatty acid composition of the glyceride fractions to become 47 area% and above is shortened compared to the case where the partial glyceride lipase is not used.

3. The method according to claim 1 or 2, wherein the partial glyceride lipase is a *Penicillium camenberti* derived lipase.

4. The method according to any one of claims 1 to 3, wherein the amount in which the *Candida cylindracea* derived lipase is used ranges from 200 to 1,600 units per gram of the feedstock oil.

5. The method according to any one of claims 1 to 4, wherein the amount in which the partial glyceride lipase is used ranges from 0.1 to 1 in terms of activity ratio relative to the *Candida cylindracea* derived lipase which is taken as 1.

6. The method according to any one of claims 1 to 5, wherein the lipase hydrolysis reaction is performed at between 10 and 30°C.

7. The method according to any one of claims 1 to 6, wherein the proportion of docosahexaenoic acid in fatty acid composition of the glyceride fractions after the reaction is no less than 45 area%.

8. The method according to any one of claims 1 to 7, wherein the proportion of eicosapentaenoic acid in fatty acid composition of the glyceride fractions after the reaction is no less than 5 area%.

9. The method according to any one of claims 1 to 8, wherein the amount of water to be added to the reaction solution after the lipase hydrolysis reaction ranges from 0.2 to 1.2 g per gram of the feedstock oil.

10. The method according to any one of claims 1 to 9, wherein the reaction is terminated at the point in time when the acid value of the reaction solution after the lipase hydrolysis reaction has come to lie between 90 and 150.

11. The method according to any one of claims 1 to 10 which further comprises a step of recovering the glycerides after the reaction.

## Fig. 1

## Fig. 2

## INTERNATIONAL SEARCH REPORT

| International application No. |
|---|
| PCT/JP2019/034729 |

### A. CLASSIFICATION OF SUBJECT MATTER
Int.Cl. C12P7/64(2006.01)i

According to International Patent Classification (IPC) or to both national classification and IPC

### B. FIELDS SEARCHED

Minimum documentation searched (classification system followed by classification symbols)
Int.Cl. C12P7/64

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/MEDLINE/WPIDS/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2012/118173 A1 (NIPPON SUISAN KAISHA, LTD.) 07 May 1998, claims, page 7, lines 8-10, examples 4, 5 (Family: none) | 1-11 |
| Y | WO 2012/118173 A1 (NIPPON SUISAN KAISHA, LTD.) 01 December 2011, claims, paragraph [0017], examples & US 2013/0123525 A1, claims, paragraph [0048], examples & EP 2578691 A1 | 1-11 |

☒ Further documents are listed in the continuation of Box C. ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13 November 2019 (13.11.2019) | 26 November 2019 (26.11.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2019/034729

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2012/118173 A1 (NIPPON SUISAN KAISHA, LTD.) 07 September 2012, claims, paragraphs [0016], [0017], production examples & US 9029584 B2, claims, column 6, lines 11-41, production examples & EP 2682453 A1 | 1-11 |
| Y | ADACHI, S. et al., "Acidolysis of sardine oil by lipase to concentrate eicosapentaenoic and docosahexaenoic acids in glycerides", J. Ferment. Bioeng., 1993, vol. 75, no. 4, pp. 259-264, in particular, summary, page 259, paragraph [0003], page 261, paragraph [0002], fig. 3 | 1-11 |
| A | JP 07-051075 A (NIPPON OIL & FATS CO., LTD.) 28 February 1995 (Family: none) | 1-11 |
| A | PINSIRODOM, P. et al., "Critical temperature for production of MAG by esterification of different FA with glycerol using Penicillium camembertii lipase", JAOCS, 2004, vol. 81, no. 6, pp. 543-547 | 1-11 |
| A | HALLDORSSON, A. et al., "Lipase selectivity toward fatty acids commonly found in fish oil", Eur. J. Lipid Sci. Technol., 2004, vol. 106, pp. 79-87 | 1-11 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S58165796 A
- JP H7268382 A
- WO 9818952 A

**Non-patent literature cited in the description**

- Japanese Pharmacopoeia
- JOCS Standard Methods for the Analysis of Fats, Oils and Related Materials. Japan Oil Chemists' Society, 2013